**Europäisches Patentamt**

**European Patent Office**

**Office européen des brevets**

(19)

(11) Veröffentlichungsnummer: **0 010 797**
**A1**

(12)

# EUROPÄISCHE PATENTANMELDUNG

(21) Anmeldenummer: 79200578.7

(22) Anmeldetag: **10.10.79**

(51) Int. Cl.³: **A 61 B 6/00, G 01 N 23/06**

---

(30) Priorität: **14.10.78 DE 2844927**

(43) Veröffentlichungstag der Anmeldung: **14.05.80**
**Patentblatt 80/10**

(84) Benannte Vertragsstaaten: **DE FR GB IT NL SE**

(71) Anmelder: **Philips-Patentverwaltung GmbH,**
**Steindamm 94, D-2000 Hamburg 1 (DE)**
(84) Benannte Vertragsstaaten: **DE**

(71) Anmelder: **N.V. Philips' Gloeilampenfabrieken,**
**Emmasingel 29, NL-5611 AZ Eindhoven (NL)**
(84) Benannte Vertragsstaaten: **FR GB IT NL SE**

(72) Erfinder: **Wagner, Wolfgang, Bockholtstwiete 11,**
**D-2000 Hamburg 63 (DE)**

(74) Vertreter: **Hartmann, Heinrich et al, Philips**
**Patentverwaltung GmbH Steindamm 94, D-2000**
**Hamburg 1 (DE)**

---

(54) **Verfahren und Vorrichtungen zur Ermittlung des Körperrandes zur Rekonstruktion einer Absorptionsverteilung in einem ebenen Untersuchungsbereich dieses Körpers.**

(57) Zur Ermittlung des Randes eines Körpers und Rekonstruktion der Absorptionsverteilung von Strahlung in einem ebenen Untersuchungsbereich wird der Körper mit einem Begrenzungskörper in Kontakt gebracht, den der Körper an unterschiedlichen in der durchstrahlten Ebene liegenden Punkten berührt. Auf jedem Strahlenweg, der durch einen der Berührungspunkte und den Untersuchungsbereich verläuft, wird ein Randpunkt des Körpers ermittelt, dessen Abstand von dem Berührungspunkt als ein Wert bestimmt wird, der dem Quotienten aus zum jeweiligen Strahlenweg gehörenden, durch Messung der Körperabsorption ermittelnden Absorptionswert $[Q(p, \vartheta)]$ und einem vorgewählten, mittleren Absorptionskoeffizienten ($\bar{\mu}$) entspricht. Die Rekonstruktion der Absorptionsverteilung im Untersuchungsbereich erfolgt derart, daß der außerhalb der den Untersuchungsbereich durchsetzenden Strahlenwege liegende Körperbereich in Streifen unterteilt wird, deren Länge durch den Körperrand begrenzt ist, wobei den Streifen Absorptionsdaten zugeordnet werden, aus denen zusammen mit den Absorptionswerten die Absorptionsverteilung im Untersuchungsbereich rekonstruiert wird.

PHD 78-137 EP

Verfahren zur Ermittlung des Körperrandes zur Rekonstruktion einer Absorptionsverteilung in einem ebenen Untersuchungsbereich eines Körpers

Die Erfindung bezieht sich auf ein Verfahren zur Ermittlung des Randes eines Körpers zur Rekonstruktion einer Absorptionsverteilung von Strahlung in einem ebenen Untersuchungsbereich des Körpers, wobei der Untersuchungsbereich in unterschiedlichen in der Ebene liegenden Richtungen auf jeweils einer Anzahl wenigstens annähernd parallel liegender Strahlenwege vollständig von Meßstrahlen zur Bestimmung von Absorptionswerten durchstrahlt wird, aus denen mit Hilfe von Randpunkten des Körpers die Absorptionsverteilung im Untersuchungsbereich rekonstruierbar ist, sowie auf eine Vorrichtung zur Durchführung des Verfahrens.

Ein derartiges Verfahren ist bereits aus dem Aufsatz "Reconstruction from truncated scan data" von W. Wagner, erschienen in Medita, Sonderheft I/78, bekannt. Mit diesem Verfahren ist es möglich, die Absorptionsverteilung von Strahlung, z.B. Röntgenstrahlung, in einem in der durchstrahlten Ebene (Untersuchungsebene) liegenden Untersuchungsbereich eines z.B. menschlichen Körpers zu rekonstruieren. Der Untersuchungsbereich kann dabei ganz oder teilweise innerhalb der durchstrahlten Körperebene liegen, wenn beispielsweise nur einzelne Organe des Körpers untersucht werden sollen. Das heißt, der Untersuchungsbereich kann einen wesentlich kleineren Durchmesser aufweisen als der in der Ebene liegende Lagerungsbereich zur Aufnahme des Körpers. Der außerhalb des Untersuchungsbereichs liegende Körperbereich wird hierbei im Gegensatz zum Untersuchungsbereich nicht in jeder Richtung vollständig von Strahlung durchsetzt, so daß die Strahlenbelastung des Körpers reduziert ist.

Um Fehler in der rekonstruierten Absorptionsverteilung des Untersuchungsbereiches zu vermeiden, müssen zusätzlich in den außerhalb des Untersuchungsbereiches liegenden Körperbereichen, die nicht vollständig von den Meßstrahlen durchsetzt werden, Absorptionsdaten ($\overline{Q}(p,\vartheta)$) ermittelt werden, die den Absorptionswerten ($Q(p,\vartheta)$) entsprechen. Hierzu ist aber wenigstens eine Kenntnis des Körperrandes erforderlich. Dieser wird, wie auch in der älteren Patentanmeldung P 28 02 593.6 beschrieben, mit Hilfe von zusätzlichen Strahlenquellen ermittelt, die eine vom Körper stark oder völlig absorbierte, in der Ebene liegende Hilfsstrahlung aussenden. Die Hilfsstrahlung wird unmittelbar in den an den Untersuchungsbereich angrenzenden Bereich emittiert, wobei sie den Lagerungsbereich durchsetzt. Ein Teil der Hilfsstrahlung läuft hierbei am Körper vorbei und trifft auf eine in der Ebene liegende Reihe von Hilfsdetektoren, mit deren Hilfe der Abstand der den Körper tangierenden Hilfsstrahlen vom Untersuchungsbereich ermittelt wird. Durch Drehung der Anordnung Hilfsstrahlenquelle-Hilfsdetektoren um den Körper herum, wird somit eine Vielzahl von den Körper tangierenden Hilfsstrahlen in unterschiedlichen Richtungen erzeugt. Der Rand des Körpers wird dann wenigstens näherungsweise als Envelope der tangierenden Hilfsstrahlen bestimmt.

Die zur Rekonstruktion der Absorptionsverteilung des Untersuchungsbereichs zusätzlich benötigten Absorptionsdaten werden derart bestimmt, daß für jede Richtung die außerhalb des Untersuchungsbereichs liegenden, nicht von den Meßstrahlen durchsetzten Bereiche des Körpers in Streifen unterteilt werden, die wenigstens annähernd parallel zu den Meßstrahlen verlaufen und deren Breite wenigstens ungefähr der der Strahlenwege, auf denen die Meßstrahlen verlaufen, entspricht. Die Länge eines Streifens ist hierbei durch den Körperrand begrenzt. Jedem Streifen wird nun ein vorgewählter, auf eine Einheitslänge bezogener Absorptionskoeffizient zugeordnet, der wenigstens annähernd die mittlere Körperabsorption beschreibt. Durch Multiplikation des vorgewählten Absorptionskoeffizienten mit jeweils einer Streifenlänge werden dann Absorptionsdaten erzeugt, die zur entsprechenden Streifenlänge proportional sind.

Mit den erhaltenen Absorptionsdaten, die so behandelt werden, als wären sie aus einer Messung gewonnen, und den Absorptionswerten wird dann, wie auch in der schon erwähnten älteren Patentanmeldung P 28 02 593.6 beschrieben, die Absorptionsverteilung bei erheblich verminderter Strahlenbelastung des Körpers rekonstruiert.

Die Installation von Hilfsstrahlenquellen bzw. von die Hilfsstrahlen messenden Hilfsdetektoren in Tomographiegeräte zur Ermittlung des Körperrandes erfordert aber einen zusätzlichen technischen Aufwand, so daß sich die Systemkosten für derartige Geräte erhöhen.

Aufgabe der Erfindung ist es, ein Verfahren und eine Vorrichtung zur Durchführung des Verfahrens anzugeben, mit dem der Rand eines Körpers unter Verwendung von Zusatzeinrichtungen bestimmbar ist, deren Installation in Tomographiegeräte einen wesentlich geringeren technischen Aufwand erfordert.

Diese Aufgabe wird erfindungsgemäß dadurch gelöst, daß der zu untersuchende Körper mit einem Begrenzungskörper in Kontakt gebracht wird, den der Körper an einer Anzahl von wenigstens annähernd in der Ebene liegenden, vorgegebenen oder durch Messung ermittelten Punkten berührt, und daß auf jedem Strahlenweg, der durch einen der berührten Punkte und den Untersuchungsbereich verläuft, ein Randpunkt ermittelt wird, dessen Abstand von dem berührten Punkt als ein Wert bestimmt wird, der dem Quotienten aus dem zum jeweiligen Strahlenweg gehörenden Absorptionswert und einem vorgewählten, mittleren Absorptionskoeffizienten entspricht.

Wird der zu untersuchende Körper mit einem Begrenzungskörper in Kontakt gebracht, so berührt der zu untersuchende Körper diesen an einer Anzahl von Punkten (Berührungspunkten), deren Koordinaten auf dem Begrenzungskörper vorgegeben sind und die in der durchstrahlten Ebene (Untersuchungsebene) des Körpers liegen. Beim Durchstrahlen des Körpers mit parallel oder fächerförmig verlaufenden Meßstrahlen - beide Strahlengeometrien können in bekannter Weise ineinander umgewandelt werden - verlaufen durch jeden Berührungspunkt eine Vielzahl von Meßstrahlen in unterschiedlichen, in der Untersuchungsebene liegenden Richtungen und durch den Untersuchungsbereich hindurch. Auf allen durch die Berührungspunkte verlaufenden Strahlenwegen, auf denen die Meßstrahlen liegen, deren Breite durch die der Strahlenwege bestimmt ist, wird jeweils ein weiterer sog. Randpunkt ermittelt, dessen Abstand von dem Berührungspunkt in Richtung des Körpers einem Quotienten $(L(p,\vartheta))$ entspricht, der sich aus einem Absorptionswert, der für jeden Strahlenweg ermittelt wird, dividiert durch einen mittleren, vorgewählten Absorptionskoeffizienten ergibt. Auf diese Weise wird der Rand des zu untersuchenden Körpers wenigstens annähernd durch die ermittelten Randpunkte bestimmt.

Es liegt im Rahmen der Erfindung, daß das Verfahren nicht nur bei einfachen Körpern, z.B. menschlichen Körpern, sondern auch bei zusammengesetzten Körpern anwendbar ist, beispielsweise bei einem menschlichen Körper, der wenigstens teilweise mit einem Wassersack umgeben ist.

Da der Begrenzungskörper in relativ einfacher Weise mit dem zu untersuchenden Körper in Kontakt gebracht werden kann - der Begrenzungskörper muß lediglich in definierter Lage zu einem Bezugssystem, z.B. dem Zentrum des Untersuchungsbereichs positioniert sein - , erfordert dieses Verfahren praktisch nur geringe Eingriffe in den Aufbau von Tomographiegeräten.

Nach einer vorteilhaften Weiterbildung der Erfindung wird auf den durch die so ermittelten Randpunkte hindurchlaufenden Strahlenwegen, ausgehend von den Randpunkten in Richtung des Körpers jeweils ein weiterer Randpunkt ermittelt, dessen Abstand von dem ursprünglichen Randpunkt als ein Wert bestimmt wird, der dem Quotienten aus dem zum jeweiligen Strahlenweg gehörenden Absorptionswert und einem vorgewählten, mittleren Absorptionskoeffizienten entspricht.

Die ursprünglich ermittelten Randpunkte stellen praktisch neue "quasi-Berührungspunkte" dar, durch die jeweils eine Vielzahl von in der Ebene liegenden Strahlenwegen verläuft. Auf jedem dieser Strahlenwege wird nun ein weiterer Berührungspunkt ermittelt, dessen Abstand von dem "quasi-Berührungspunkt" in Richtung des Körpers wiederum dem für den entsprechenden Strahlenweg ermittelten Quotienten ($L(p,\vartheta)$) entspricht. Hierdurch wird erreicht, daß die Zahl der den Körperrand wenigstens näherungsweise beschreibenden Randpunkte erheblich gesteigert wird.

Nach einer anderen vorteilhaften Weiterbildung der Erfindung werden zur Ermittlung verbesserter Randpunkte vom Zentrum des Untersuchungsbereichs ausgehende und bis zu den Randpunkten weisende Vektoren in vorgewählten Winkelbereichen gemittelt, wodurch der Rand des Körpers genauer bestimmt
wird.

Vom Zentrum des Untersuchungsbereichs werden hierzu zu den
Randpunkten weisende, in der Untersuchungsebene liegende
Vektoren (A(r, φ)) bestimmt, die in vorgegebenen Winkelbereichen (Δφ) nach bekannten Verfahren gemittelt werden,
so daß für jeweils ein Winkelbereich ein den Körperrand
repräsentierender, verbesserter Randpunkt zur Verfügung
steht.

Nach einer weiteren vorteilhaften Ausbildung der Erfindung
besitzt eine Vorrichtung zur Durchführung des Verfahrens
wenigstens eine Strahlenquelle, deren Strahlung den auf
einem Untersuchungstisch liegenden Körper im Untersuchungsbereich in unterschiedlichen in der Ebene liegenden Richtungen auf jeweils einer Anzahl wenigstens annähernd
parallel liegender Strahlenwege vollständig durchstrahlt,
wobei die Strahlung zur Aufnahme von Meßwerten auf eine
Detektoranordnung trifft, welche jenseits des Körpers angeordnet ist, sowie eine elektronische Einheit zur Ermittlung
der Absorptionsverteilung der Strahlung mit Hilfe der Meßwerte und der Randpunkte, wobei ein auf dem Patiententisch in definierter Lage befestigbarer, aus starrem
Material bestehender Begrenzungskörper vorgesehen ist, auf
dem an vorgegebenen Positionen einzelne, wenigstens
annähernd in der Ebene liegende Signalgeber angeordnet
sind, die bei Berührung mit dem Körper ein Ausgangssignal
erzeugen. Der Begrenzungskörper ist derart ausgebildet,
daß er in definierter Lage auf einem Patiententisch montierbar ist, so daß bereits fertiggestellte Tomographiegeräte
sich mit diesem Begrenzungskörper zusätzlich ausrüsten
lassen.

Die einzelnen mit dem Begrenzungskörper verbundenen Signalgeber liegen auf der dem zu untersuchenden Körper zugewandten

PHD 78-137 EP

Seite an vorgegebenen Positionen des Begrenzungskörpers
sowie in der Untersuchungsebene. Sie erzeugen bei Berührung
mit dem Körper jeweils ein Ausgangssignal, woraus die Lage
des Signalgebers ermittelt wird.

Nach einer vorteilhaften Weiterbildung der Erfindung besteht der Begrenzungskörper aus einer ebenen, rechteckförmigen Platte und zwei Begrenzungsplatten, welche an
der Platte an gegenüberliegenden Seiten um jeweils eine
Achse schwenkbar angeordnet sind, wobei die Achsen senkrecht zur Ebene verlaufen.

Durch die plattenförmige Ausbildung des Begrenzungskörpers
wird erreicht, daß dieser in besonders einfacher Weise
herstellbar und auf einem Patiententisch eines Tomographiegerätes positionierbar ist. Die beiden Begrenzungsplatten,
die wie die rechteckförmige Platte aus starrem Material
bestehen, bewirken zusätzlich eine Erhöhung der Zahl
der Berührungspunkte, indem sie an die Seiten des zu
untersuchenden Körpers angelegt werden. Auf diese Weise
ist eine genauere Bestimmung des Körperrandes möglich.

Nach einer weiteren vorteilhaften Ausbildung der Erfindung
sind auf dem Begrenzungskörper mehrere Zeilen von parallel
zur Ebene liegenden Signalgebern angeordnet, wodurch
erreicht wird, daß bei Durchstrahlung des zu untersuchenden
Körpers in mehreren parallel zueinander liegenden Untersuchungsebenen der Begrenzungskörper relativ zum Körper
nicht bewegt zu werden braucht.

Nach einer anderen vorteilhaften Ausbildung der Erfindung
besitzt eine Vorrichtung zur Durchführung des Verfahrens
wenigstens eine Strahlenquelle, deren Strahlung den auf
einem Untersuchungstisch liegenden Körper im Untersuchungsbereich in unterschiedlichen in der Ebene liegenden Richtungen auf jeweils einer Anzahl wenigstens annähernd parallel liegender Strahlenwege vollständig durchstrahlt, wobei die Strahlung zur Aufnahme von Meßwerten auf eine Detektoranordnung trifft, welche jenseits des Körpers angeordnet ist, sowie eine elektronische Einheit zur Ermittlung der Absorptionsverteilung der Strahlung mit Hilfe der Meßwerte und der Randpunkte, wobei ein auf dem Patiententisch in definierter Position befestigbarer, aus starrem Material bestehender Begrenzungskörper mit vorgegebener Geometrie vorgesehen ist, der mit einer flexiblen Folie überdeckt ist, und daß sich zwischen der Folie und dem Begrenzungskörper eine Flüssigkeit befindet, deren Absorptionskoeffizient wenigstens annähernd dem des Körpers entspricht.

Mit Hilfe eines derartigen Begrenzungskörpers kann die Zahl der Berührungspunkte erheblich gesteigert werden, da bei vorgegebener Geometrie des Berührungskörpers alle Berandungspunkte der dem Körper zugewandten Seite des Begrenzungskörpers als Berührungspunkte angesehen werden können. Hierzu liegt die flexible Folie, zwischen der und dem Begrenzungskörper sich die Flüssigkeit befindet, eng am Körper an. Der zu untersuchende Körper bildet daher mit der ihn wenigstens teilweise umgebenden Flüssigkeit einen zusammengesetzten Untersuchungskörper, der dem zu untersuchenden Körper entspricht.

Die Zeichnung stellt Ausführungsbeispiele der Erfindung dar. Es zeigen

Fig. 1 ein Röntgentomographiegerät,

Fig. 2 einen zu untersuchenden Körper mit einem Begrenzungskörper,

Fig. 3 einen Begrenzungskörper in perspektivischer Darstellung,

Fig. 4 ein Kreuzleitersystem zur Bildung einzelner Signalgeber sowie ein zugehöriges Blockschaltbild zum Verarbeiten der durch die Signalgeber erzeugten Signale,

Fig. 5 einen Ausschnitt aus dem Begrenzungskörper mit einem Winkelgeber,

Fig. 6 ein Blockschaltbild einer elektronischen Einheit zur Durchführung des Verfahrens,

Fig. 7 einen in der Untersuchungsebene liegenden Körperschnitt,

Fig. 8 ein Diagramm zur Darstellung der vom Zentrum des Untersuchungsbereichs in den Randpunkten verlaufenden Detektoren in Abhängigkeit eines in der Untersuchungsebene liegenden Winkels φ zur Ermittlung verbesserter Randpunkte,

Fig. 9 einen in der Untersuchungsebene liegenden Schnitt durch einen mit einer flexiblen Folie überdeckten Begrenzungskörper,

Fig. 10 einen in der Ebene liegenden Schnitt durch einen mit einer flexiblen Folie überdeckten Begrenzungskörper, auf dem ein zu untersuchender Körper liegt, und

Fig. 11 eine perspektivische Ansicht eines mit einer flexiblen Folie überdeckten Begrenzungskörper.

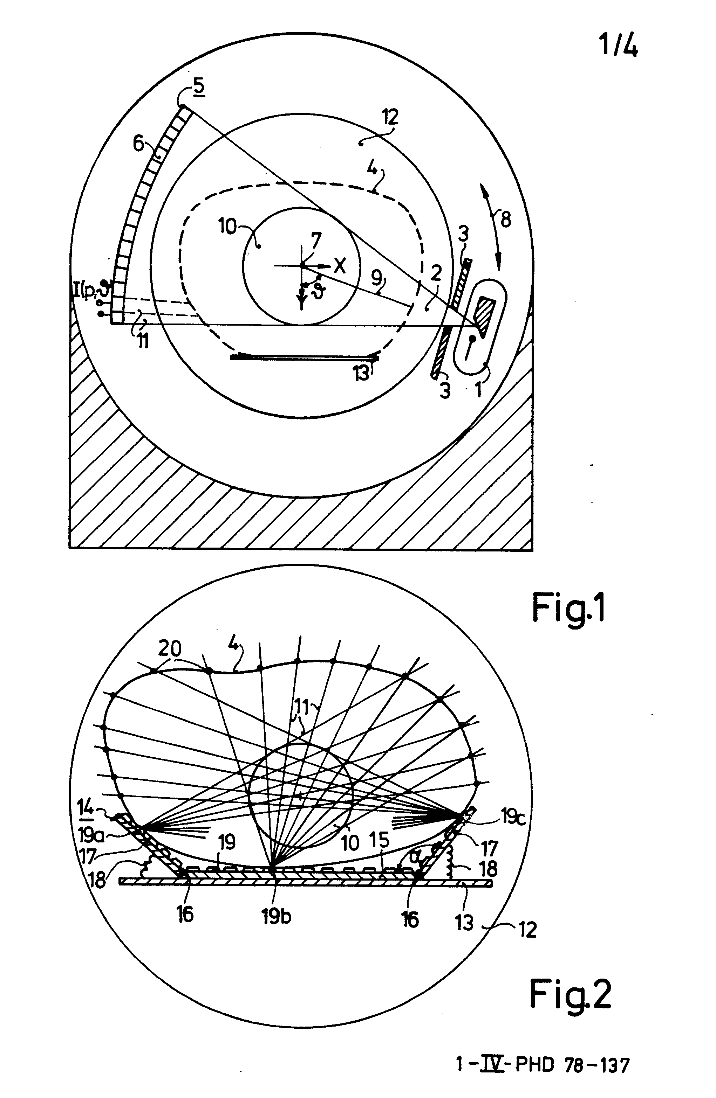

Fig. 1 zeigt einen Schnitt durch ein schematisch dargestelltes Röntgentomographiegerät, welches aus einer Strahlenquelle 1 zum Aussenden eines fächerförmigen Röntgenstrahlenbündels 2 besteht, das in der Schnittebene (Zeichenebene), die die Untersuchungsebene darstellt, verläuft, und das mittels einer Bleiblende 3 begrenzt ist. Das Röntgenstrahlenbündel 2 durchdringt einen zu untersuchenden Körper 4 und trifft auf eine Detektorreihe 5, welche aus einzelnen in der Untersuchungsebene nebeneinander liegenden Strahlungsdetektoren 6 besteht. Das System Strahlenquelle 1 - Detektorreihe 5 ist um eine Zentralachse 7, die senkrecht zur Untersuchungsebene verläuft, in Pfeilrichtung 8 drehbar angeordnet, wobei seine Stellung relativ zu einem in der Untersuchungsebene liegenden, rechtwinkligen Koordinatensystem X,Y durch einen Drehwinkel $\vartheta$, den der Zentralstrahl 9 des fächerförmigen Strahlenbündels 2 mit der Y-Achse einschließt, angegeben wird. Der Ursprung des Koordinatensystems X,Y, durch den die Zentralachse 7 hindurchläuft,
ist gleichzeitig Zentrum des Untersuchungsbereichs 10 des
Röntgentomographiegerätes. Dies ist der in der Untersuchungsebene liegende Bereich, der unter jedem Drehwinkel $\vartheta$ vollständig von auf Strahlenwegen 11 verlaufenden Meßstrahlen
durchstrahlt wird, wobei die Breite der Strahlenwege 11
durch die Breite der Detektoren 6 bestimmt ist. Zur Lagerung
des zu untersuchenden Körpers 4 (gestrichelt gezeichnet),
der in einem den Untersuchungsbereich 10 konzentrisch
umgebenden Lagerungsbereich 12 liegt, ist ein senkrecht
zur Untersuchungsebene verstellbarer Patiententisch 13
vorgesehen. Seine mechanische Halterung ist der Übersicht
wegen nicht dargestellt.

Durch eine Veränderung der Lage des Körpers 4 innerhalb des
Lagerungsbereichs 12 kann erreicht werden, daß der Untersuchungsbereich 10, der in seiner Größe durch Verstellen
der Blende 3 veränderbar ist, verschiedene zu diagnostizierende Bereiche innerhalb des Körpers 4 überdeckt. Hierzu
muß natürlich zwischen dem Körper 4 und dem Lagerungsbereich 12 genügend Spiel sein.

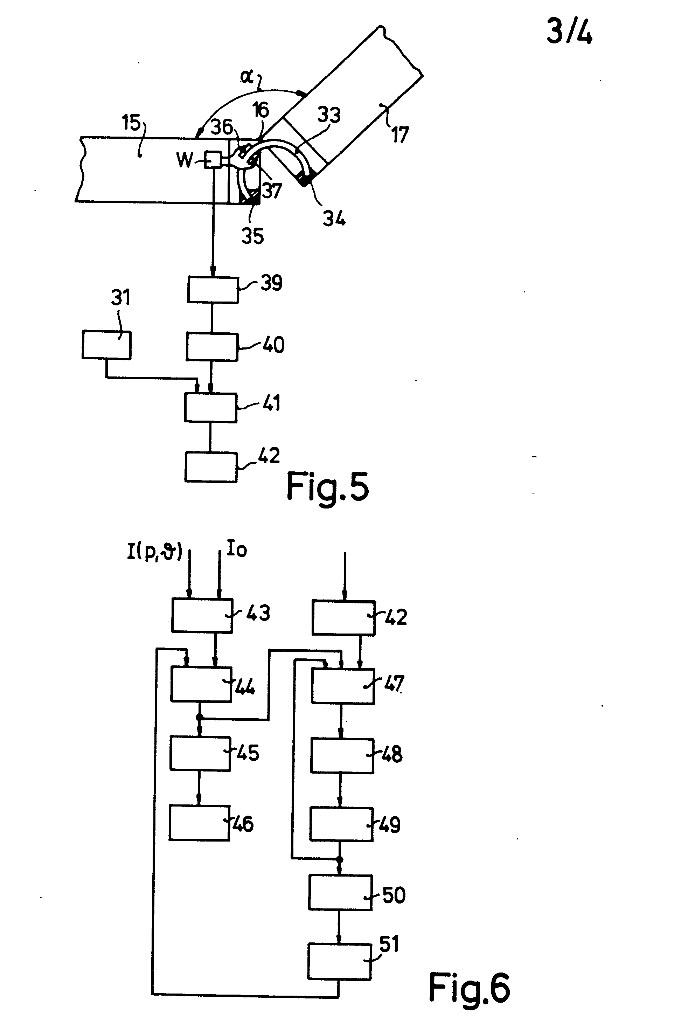

In der Fig. 2 liegt der zu untersuchende Körper 4 auf einem
Begrenzungskörper 14, der in definierter Lage auf dem
Patiententisch 13 befestigt ist. Der Begrenzungskörper 14
ist hierbei aus unverbiegbarem und wenigstens annähernd
röntgenstrahlendurchlässigem Material gefertigt und besteht
aus einer rechteckförmigen Platte 15, an der an gegenüberliegenden Seiten jeweils um eine Achse 16 schwenkbare Begrenzungsplatten 17 angeordnet sind, die ebenfalls rechteckförmig ausgebildet sind und mit Hilfe von Federn 18 an
den Körper 4 gedrückt werden. Der zwischen der Platte 15
und den Begrenzungsplatten 17 auftretende Winkel $\alpha$ wird
mittels eines Winkelaufnehmers (siehe Fig. 5) gemessen.
Die Achsen 16 verlaufen senkrecht zur Untersuchungsebene, die in Fig. 1 und 2 die Zeichenebene ist. Anstelle der Federn 18 können auch Keile aus elastischem Schaumstoff verwendet werden, oder die Begrenzungsplatten 17 werden mit Hilfe eines über den Körper 4 spannbaren elastischen Seils verbunden.

Auf dem Begrenzungskörper 14 befinden sich einzelne Signalgeber 19, die hier nur schematisch dargestellt sind, und die an vorgegebenen Orten in der Untersuchungsebene liegen. Bei Berührung mit dem Körper 4 erzeugen die Signalgeber 19 jeweils ein Ausgangssignal, z.B. ein elektrisches Ausgangssignal, so daß hierdurch der vom Körper 4 berührte Signalgeber 19 identifiziert und dadurch dessen Ort in der Untersuchungsebene bestimmt ist. Es ist selbstverständlich, daß die Begrenzungsplatten 17 so stark an den Körper 4 angedrückt werden, daß die auf ihnen positionierten Signalgeber 19 ansprechen.

Berührt der Körper 4, wie in Fig. 2 dargestellt, den Begrenzungskörper 14 an drei Berührungspunkten bzw. an drei Signalgebern 19a-c, so werden alle Strahlenwege, z.B. die Strahlenwege 11, ermittelt, die durch die Signalgeber 19a-c und den Untersuchungsbereich 10 verlaufen. Die einzelnen Strahlenwege 11 werden hierbei durch umgerechnete Koordinaten $(p, \vartheta)$ einer ebenen parallelen Strahlengeometrie beschrieben, wobei $\vartheta$ der Drehwinkel ist, den ein beliebiger Strahlenweg 11 mit der Y-Achse des Koordinatensystems XY einschließt, und wobei p den senkrechten Abstand des Strahlenweges vom Zentrum des Koordinatensystems XY angibt. Es ist bekannt, daß eine derartige parallele Strahlengeometrie durch Umsortierung von Strahlenwegen einer fächerförmigen Strahlengeometrie (Strahlenbündel 2) ermittelt werden kann.

Aus den mit Hilfe der Detektoren 6 gewonnenen Meßwerten $I(p, \vartheta)$ entlang der jeweiligen Strahlenwege 11 werden dann

Absorptionswerte $Q(p,\vartheta)$ für jeden Strahlenweg 11 gebildet, die zu

$$Q(p,\vartheta) = -\ln \left\{ I(p,\vartheta)/I_0 \right\} \tag{1}$$

bestimmt werden. Hierbei ist $I_0$ die nicht vom Körper 4 geschwächte Strahlintensität, die von Referenzdetektoren gemessen wird.

Die für jeweils einen Strahlenweg 11 erhaltenen Absorptionswerte $Q(p,\vartheta)$ werden durch einen vorgewählten Körperabsorptionswert $\bar{\mu}$ (mittlerer Absorptionskoeffizient) dividiert, der auf eine Einheitslänge bezogen ist, so daß auf diese Weise ein Quotient $L(p,\vartheta)$ gebildet wird, der einer tatsächlichen Länge entspricht. Der Körperabsorptionswert $\bar{\mu}$ ist zweckmäßigerweise der vom Wasser bei der eingestellten Energie der Röntgenstrahlen.

Auf jeweils einem Strahlenweg 11 wird nun, ausgehend von den entsprechenden Signalgebern 19a-c (Berührungspunkten) und in Richtung des Körpers 4 ein Punkt bestimmt, der einen Randpunkt 20 des Körpers 4 darstellt und dessen Abstand vom jeweiligen Signalgeber 19a-c dem Quotienten $L(p,\vartheta)$ entspricht. Wie in Fig. 2 gezeigt, überlagern sich einige der Randpunkte 20. Je mehr Überlagerungen, d.h. je mehr Signalgeber 19 den Körper 4 berühren, desto genauer ist der tatsächliche Rand des Körpers 4 durch Mittelung der Randpunkte 20 zu bestimmen. Auf die Mittelung der Randpunkte wird weiter unten eingegangen.

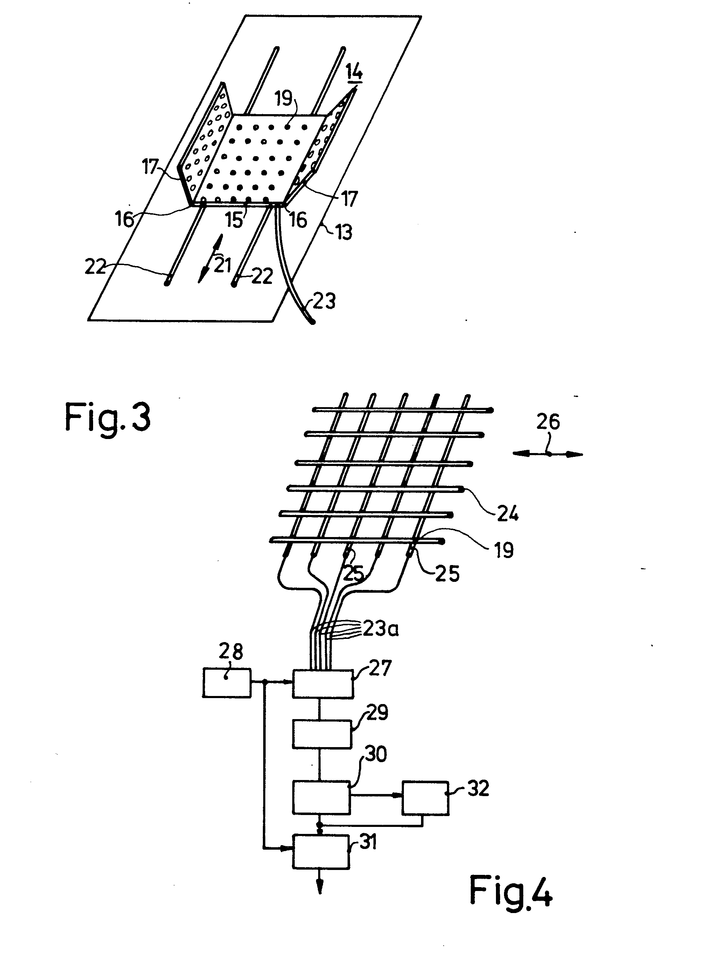

Fig. 3 stellt eine perspektivische Ansicht des Begrenzungskörpers 14 dar. Die rechteckförmige Platte 15 sowie die Begrenzungsplatten 17 haben eine Ausdehnung in Längsrichtung 21 des Patiententisches 13 von vorzugsweise etwa 10 cm, so daß mehrere Zeilen von Signalgebern 19 parallel zur Untersuchungsebene auf dem Begrenzungskörper 14 angeordnet werden können. Dies ist erforderlich, da üblicherweise nacheinander mehrere benachbarte Ebenen des Körpers durchstrahlt werden. Der Zeilenabstand der Signalgeber 19 entspricht hierbei dem Abstand der zu durchstrahlenden Ebenen. Der Körper 4 (Patient) braucht somit relativ zum Begrenzungskörper 14 nicht verschoben zu werden. Andererseits muß der Begrenzungskörper 14 in Längsrichtung 21 des Patiententisches 13 verstellbar sein, damit unterschiedliche, weit auseinanderliegende Gebiete des Körpers abgetastet werden können.

Zu diesem Zweck sind auf dem Patiententisch 13 Kunststoffschienen 22 fest aufgebracht, die in Längsrichtung 21 liegen, und die in entsprechende, auf der Unterseite der Platte 15 eingebrachte Nuten eingreifen. Die Kunststoffschienen sind mit einer Skaleneinteilung versehen, um eine reproduzierbare Einstellung des Begrenzungskörpers 14 durch das Bedienungspersonal zu ermöglichen.

Die elektrischen Signale der Signalgeber 19 werden über ein Kabel 23 an eine weiter unten zu erläuternde elektronische Einheit geleitet.

Ferner kann der Patiententisch 13 auch so ausgebildet sein, daß dieser im Bereich der Untersuchungsebene unterbrochen ist, so daß die Röntgenstrahlung nicht zusätzlich von ihm geschwächt wird.

In Fig. 4 wird eine beispielsweise Ausführungsform der einzelnen Signalgeber 19 dargestellt. Sie sind als Kreuzungspunkte von parallelen Quer- 24 und Längsleiterbahnen 25 definiert, die untereinander den gleichen Abstand haben und senkrecht zueinander verlaufen. Die Querleiterbahnen 24 verlaufen hierbei parallel zur Untersuchungsebene und sind, wie die Längsleiterbahnen 25, auf jeweils einer Seite einer dünnen, aus Kunststoff bestehenden Folie aufgedampft, die durch eine dünne, röntgendurchlässige Schaumstoffschicht
(nicht dargestellt) auf Abstand gehalten werden, derart,
daß die bedampften Seiten der Folien einander zugewandt
sind. Die Schaumstoffschicht ist im Bereich der Kreuzungspunkte (Signalgeber 19) mit Löchern versehen, so daß bei
Druckbelastung der Signalgeber 19 zwischen den Quer- 24
und den Längsleiterbahnen 25, die z.B. aus einem Röntgenstrahlung nur wenig absorbierenden Metall bestehen, ein
direkter Kontakt auftritt. Beispielsweise können sie
aus mit einer dünnen Goldschicht bedeckten Aluminiumbahnen bestehen.

Die Schaumstoffschicht kann auch durchgehend ausgebildet
sein und eine schwache elektrische Leitfähigkeit besitzen,
die sich bei Druckbelastung stark vergrößert. Beide Ausführungsformen der Schaumstoffschichten erlauben eine
einfache und kostengünstige Herstellung von Signalgebern 19.

Der Ort der Signalgeber 19 auf der Platte 15 bzw. auf den
Begrenzungsplatten 17 ist, wie schon erwähnt, vorgegeben,
so daß zur Bestimmung der Ortskoordinaten der Signalgeber 19,
die vom Körper 4 tatsächlich berührt werden, deren Ausgangssignale erfaßt werden. Im Betrieb wird jeweils diejenige
Querleiterbahn 24, die sich in der Untersuchungsebene
(angedeutet durch den Pfeil 26) befindet, an eine Stromversorgung angeschlossen, während die Längsleiterbahnen 25 zur
Erfassung von (Ausgangs-) Signalen ständig von einem über
die Leitungen 23a und ihnen verbundenen Analogmultiplexer 27
ausgelesen werden, der von einem Taktgenerator 28 gesteuert
wird. Die Signale werden in einem Verstärker 29 verstärkt
und in einem Analog-Digital-Wandler 30 digitalisiert. In
der einfachsten Ausführungsform ist der Analog-Digital-
Wandler 30 ein Schwellwertverstärker. Die digitalisierten
Signale werden dann in einem Speicher 31 gespeichert.

Für die auf der Platte 15 angeordneten Signalgeber 19 findet damit eine einfache Zuordnung zwischen den an den Längsleiterbahnen 25 anliegenden Signalen und den auf der X-Koordinatenachse (Fig. 1) liegenden Ortskoordinaten $x_i$ der einzelnen Signalgeber 19 statt. Die $y_i$-Koordinaten der auf der Platte 15 befindlichen Signalgeber 19 sind alle als konstant anzusehen, da die Schaumstoffschicht sehr dünn ist. Sie besitzen beispielsweise alle den gleichen Abstand von der Oberfläche des Patiententisches 13, deren Höhe durch die Ortskoordinate y beschrieben wird.

Für die Bestimmung der Ortskoordinaten der auf den Begrenzungsplatten 17 angeordneten Signalgeber 19 ist zusätzlich eine Ermittlung des Winkels α zwischen der Platte 15 und den Begrenzungsplatten 17 erforderlich.

In Fig. 5 ist ein Winkelaufnehmer dargestellt, der aus einem flexiblen und gekrümmten Kunststoffstab 33 besteht, der in bewegbaren Lagern 34, 35 gelagert ist. Oberhalb und unterhalb des Stabes 33 ist je ein zug- bzw. druckempfindliches Widerstandselement 36, 37 aufgeklebt, die zusammen mit zwei weiteren (nicht dargestellten) druckunabhängigen Widerständen zu einer elektrischen Widerstandsbrücke W zusammengeschaltet sind. Bei Drehung einer Begrenzungsplatte 17 um eine Achse 16 wird der Stab 33 auf Biegung beansprucht. Die Zug- bzw. Druckkräfte an seiner Ober- bzw. Unterseite bewirken eine Widerstandsänderung der Elemente 36, 37 und damit eine Verstimmung der Widerstandsbrücke W. Die erzeugten winkelabhängigen elektrischen Signale werden auf einen Verstärker und Analog-Digital-Wandler 39 geführt. In einem Speicher 40 ist der Zusammenhang zwischen der Widerstandsverstimmung und dem Winkel α gespeichert. Die Recheneinheit 41 berechnet aus den im Speicher 31 (Fig. 4) gespeicherten Positionen derjenigen Signalgeber 19, die sich auf den Begrenzungsplatten 17 befinden und dem Winkel α die entsprechenden winkelabhängigen Koordinaten $x_i(\alpha)$, $y_i(\alpha)$. In einem Speicher 42 werden dann sowohl

die Koordinaten $x_i$, $y_i$ der auf der Platte 15 angeordneten Signalgeber 19 als auch die Koordinaten $x_i(\alpha)$, $y_i(\alpha)$ der auf den Begrenzungsplatten 17 angeordneten Signalgeber 19 gespeichert.

Zur Erhöhung der Zahl der vom Körper 4 berührten Signalgeber 19 kann ferner eine dicke, beispielsweise 5 cm starke, nahezu röntgenstrahlendurchlässige und schwach leitfähige Schaumstoffschicht zur Anwendung kommen, die sich zwischen den Quer- 24 bzw. Längsleiterbahnen 25 befindet. Bei Druckbelastung durch den Körper 4 wird diese Schaumstoffschicht an verschiedenen Orten in der Untersuchungsebene mehr oder weniger stark komprimiert, derart, daß sie sich in weiten Bereichen an den Körper 4 anschmiegt. An den komprimierten Stellen erhöht sich ihre elektrische Leitfähigkeit, so daß die vom Körper 4 beaufschlagten Signalgeber 19 Ausgangssignale erzeugen, die von der Kompression der Schaumstoffschicht bzw. vom Abstand ihrer deformierten Oberfläche von einem Bezugssystem, beispielsweise dem Patiententisch 13, abhängen.

Die Koordinaten $x_i$, $y_i$ der Signalgeber 19 auf der Platte 15 und die Koordinaten $x_i(\alpha)$, $y_i(\alpha)$ derjenigen auf den Begrenzungskörpern 17 müssen dann entsprechend korrigiert werden. Hierzu ist der Zusammenhang zwischen der Kompression der Schaumstoffschicht (bzw. dem Abstand ihrer Oberfläche von der Tischplatte 13) und ihrer elektrischen Leitfähigkeit in einem Speicher 32 (Fig. 4) gespeichert. Die digitalen Ausgangswerte des Analog-Digital-Wandlers 30, die druckabhängig sind, werden dann zusätzlich zur Adressierung im Speicher 32 verwendet, wobei der unter jeder Adresse gefundene Abstand ebenfalls im Speicher 31 gespeichert wird.

In Fig. 6 wird das Verfahren anhand eines Blockschaltbildes erläutert. Die mit Hilfe der Detektoren 6 gemessenen Meß-

werte $I(p,\vartheta)$ (Intensitäten) bzw. die Referenzintensität $I_o$ werden in einer Logarithmiereinheit 43 logarithmiert, welche gleichzeitig Absorptionswerte $Q(p,\vartheta)$ bildet, die in einem Datenspeicher 44 gespeichert werden. Diese Absorptionswerte $Q(p,\vartheta)$ werden später benutzt, um im Zentralrechner 45 die Absorptionsverteilung $\mu(x,y)$ im Untersuchungsbereich 10 zu rekonstruieren, welche auf dem Monitor 46 dargestellt wird.

Eine erste Recheneinheit 47 sucht nun zu jeder der im Speicher 42 gespeicherten Koordinaten $x_i$, $y_i$, $x_i(\alpha)$, $y_i(\alpha)$ der vom Körper 4 berührten Signalgeber 19 die durch sie hindurchlaufenden Strahlenwege 11 und die zu den Strahlenwegen 11 gehörenden Absorptionswerte $Q(p,\vartheta)$ auf, die im Datenspeicher 44 gespeichert sind. Aus diesen Absorptionswerten $Q(p,\vartheta)$ berechnet eine zweite Recheneinheit 48 die Quotienten $L(p,\vartheta)$ und daraus Randpunkte 20 des Körpers 4, wie bereits beschrieben.

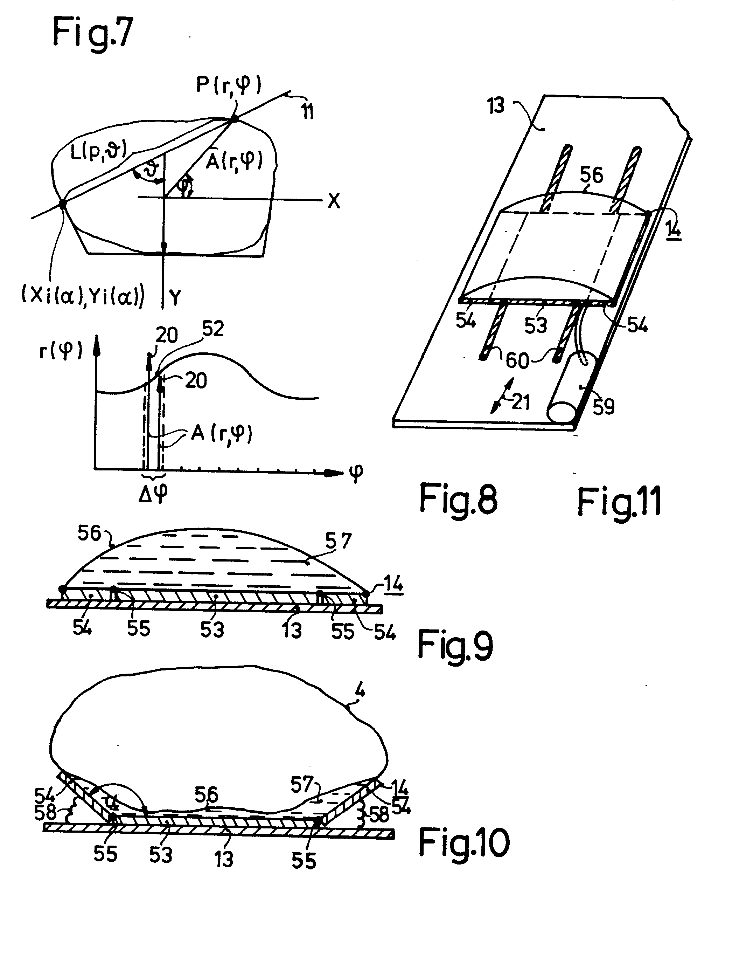

In der dritten Recheneinheit 49 erfolgt die Ermittlung weiterer Randpunkte 20 zur Erzielung einer gleichmäßigen Belegung mit ihnen entlang des Körperrandes. Zu diesem Zweck wird der Rand des Körpers 4, wie in Fig. 7 dargestellt, durch Polarkoordinaten $(r,\varphi)$ beschrieben. Das Zentrum des Polarkoordinatensystems stimmt dabei mit dem Ursprung des Koordinatensystems XY überein. In Fig. 7 verläuft ein Strahlenweg 11 beispielsweise durch die Koordinaten $x_i(\alpha)$, $y_i(\alpha)$ eines vom Körper 4 berührten Signalgebers 19, wobei auf dem Strahlenweg 11 ein Randpunkt $P(r,\varphi)$ des Körpers 4 festgelegt ist, dessen Abstand vom Signalgeber 19 dem Quotienten $L(p,\vartheta)$ entspricht. Für einen Zentralvektor $A(r,\varphi)$, der vom Zentrum des Polarkoordinatensystems, welches gleichzeitig Zentrum des Untersuchungsbereichs 10 ist, zu dem Randpunkt $P(r,\varphi)$ verläuft, gilt, wenn es sich um den i-ten Strahlenweg 11 handelt:

$$p_i = r \cos (\vartheta - \varphi).\tag{2}$$

Zusammen mit dem ermittelten Quotienten $L(p_i, \vartheta)$ folgt:

$$r^2 = x_i^2(\alpha) + y_i^2(\alpha) + 2L(p_i, \vartheta) \cdot B\tag{3}$$

mit

$$B = \left\{ x_i(\alpha) \cos(\vartheta + 90) + y_i(\alpha) \cdot \sin(\vartheta + 90) \right\}\tag{4}$$

und

$$\varphi = \text{arc } \cos(p_i/r) + \vartheta\tag{5}$$

Trägt man die Werte $r(\varphi)$ für alle derart gefundenen Vektoren $A(r, \varphi)$ gegen den Winkel $\varphi$ in einem Diagramm auf, wie in Fig. 8 gezeigt, so ergibt sich eine Belegung von diskreten Werten $r(\varphi)$, die in einigen Bereichen von $\varphi$ dichter ist und in anderen Bereichen weniger dicht ist. Um die Gleichmäßigkeit der Belegung der Randpunkte entlang des Körperrandes zu erhöhen, werden einzelne Werte $r(\varphi)$ aus den dicht belegten Bereichen von $\varphi$ ausgewählt und ihre Koordinate $x_p$, $y_p$ im Koordinatensystem XY berechnet. Diese Koordinaten $x_p$, $y_p$ werden dann wieder der ersten Recheneinheit 47 (Fig. 6) zugeführt, so daß weitere Randpunkte 20 in der schon beschriebenen Weise ermittelbar sind. Dieser Verfahrensschritt kann wenn nötig wiederholt werden.

Danach werden mit Hilfe einer in Fig. 6 dargestellten vierten Recheneinheit 50 verbesserte Randpunkte 52 ermittelt, indem über den gesamten Winkelbereich $\varphi$ die in einzelnen, begrenzten Winkelabschnitten $\delta\varphi$ liegenden Randpunkte 20 (Fig. 8) nach bekannten Verfahren gemittelt werden. Die Ausdehnung der Winkelabschnitte beträgt beispielsweise 1 Grad.

Eine in Fig. 6 dargestellte fünfte Recheneinheit 51 erstellt dann mit Hilfe des ermittelten Randes des Körpers 4 in der eingangs beschriebenen Weise die Absorptionsdaten $\overline{Q}(p, \vartheta)$, die zur Ermittlung eines Rekonstruktionsbildes des Untersuchungsbereiches 10 ebenfalls dem Datenspeicher 44 zugeführt werden.

In Fig. 9 bis 11 ist eine weitere Ausführungsform eines Begrenzungskörpers 14 dargestellt. Die Fig. 9 zeigt einen in der Untersuchungsebene liegenden Schnitt durch einen Begrenzungskörper 14, der auf dem Patiententisch 13 in definierter Weise anordbar und im unbelasteten Zustand dargestellt ist. Er ist nahezu aus röntgenstrahlendurchlässigem Kunststoff gefertigt und besteht aus einer dünnen, rechteckförmigen Auflageplatte 53 mit zwei rechteckförmigen Seitenplatten 54, welche an der Auflageplatte 53 an gegenüberliegenden Seiten um jeweils eine Achse 55 schwenkbar angeordnet sind. Die Achsen 55 verlaufen hierbei senkrecht zur Untersuchungsebene, die die Zeichenebene darstellt. Der Begrenzungskörper 14 ist mit einer flexiblen Folie 56 überdeckt, wobei sich zwischen der Folie 56 und dem Begrenzungskörper 14 eine Flüssigkeit 57 befindet, deren Absorptionskoeffizient dem mittleren Absorptionskoeffizienten des Körpers 4 (Fig. 10) entspricht. Als Flüssigkeit 57 wird vorzugsweise Wasser verwendet. Die Folie 56 ist so vorgespannt, daß sie etwa einen kissenartigen Querschnitt aufweist.

In Fig. 10 liegt auf dem Begrenzungskörper 14 ein zu untersuchender Körper 4. Die Seitenplatten 54 sind so weit wie möglich, beispielsweise mit Hilfe von Federn 58, an den Körper 4 herangedrückt. Die Folie 56 schmiegt sich hierbei an den Körper 4 an, wobei die Flüssigkeit 57 in einen Ausgleichsbehälter 59 (Fig. 11) abfließen kann, der nicht im Röntgenstrahlbereich liegt. Die Flüssigkeit 57 und der Körper 4 bilden nun für die Röntgenstrahlen einen nahezu einheitlichen, zusammengesetzten Untersuchungskörper, der sich eng an den Begrenzungskörper 14 anschmiegt. Da der Verlauf des Begrenzungskörpers 14 in der Untersuchungsebene vorgesehen ist, der Winkel $\alpha$ zwischen den Seitenplatten 54 und der Auflageplatte 53 wird in der schon unter Fig. 5 beschriebenen Weise ermittelt, sind alle Punkte auf dem Begrenzungskörper als Berührungspunkte $x_i$, $y_i$ bzw. $x_i(\alpha)$, $y_i(\alpha)$ anzusehen. Hierdurch ist es möglich, eine erheblich größere Anzahl von Randpunkten 20 des Körpers 4 in der schon beschriebenen Weise zu ermitteln.

Fig. 11 stellt eine perspektivische Ansicht des Begrenzungskörpers 14 dar. Er besitzt eine Längsausdehnung von vorzugsweise 40 cm, wobei ein mittlerer Bereich von 30 cm für die Verwendung in der Untersuchungsebene vorgesehen ist. Dies ist erforderlich, wenn nacheinander mehrere parallele Schichten des Körpers 4 durchstrahlt werden, ohne daß er zwischendurch umgelagert werden soll. Der Begrenzungskörper 14 ist ferner in Längsrichtung 21 justierbar.

Zu diesem Zweck sind Kunststoffschienen 60 fest auf dem Patiententisch 13 montiert, auf welchen der Begrenzungskörper 14, der entsprechende Nuten aufweist, gleitet. Die Schienen 60 sind mit einer Skaleneinteilung versehen, um eine reproduzierbare Einstellung des Begrenzungskörpers 14 zu ermöglichen.

Der Begrenzungskörper 14 kann in seiner Geometrie auch dem in der Untersuchungsebene liegenden Querschnitt des Körpers 4 angepaßt und z.B. schalenförmig ausgebildet sein, so daß ein Begrenzungskörper 14 ohne bewegliche Teile (z.B. Seitenplatten 54) erhalten wird.

Es bleibt zu bemerken, daß die erfindungsgemäßen Einrichtungen auch für andere Röntgentomographieanordnungen geeignet sind, beispielsweise für solche, die abweichend von Fig. 1 mit fest angeordneten, in der Untersuchungsebene liegenden und den Untersuchungsbereich z.B. kreisförmig umgebenden Detektoren versehen sind.

PATENTANSPRÜCHE:

1.    Verfahren zur Ermittlung des Randes eines Körpers zur Rekonstruktion einer Absorptionsverteilung von Strahlung in einem ebenen Untersuchungsbereich des Körpers, wobei der Untersuchungsbereich in unterschiedlichen in der Ebene liegenden Richtungen auf jeweils einer Anzahl wenigstens annähernd parallel liegender Strahlenwege vollständig von Meßstrahlen zur Bestimmung von Absorptionswerten $(Q(p,\vartheta))$ durchstrahlt wird, aus denen mit Hilfe von Randpunkten des Körpers die Absorptionsverteilung im Untersuchungsbereich rekonstruierbar ist, dadurch gekennzeichnet, daß der zu untersuchende Körper (4) mit einem Begrenzungskörper (14) in Kontakt gebracht wird, den der Körper an einer Anzahl von wenigstens annähernd in der Ebene liegenden, vorgegebenen oder durch Messung ermittelten Punkten berührt, und daß auf jedem Strahlenweg (11), der durch einen der berührten Punkte und den Untersuchungsbereich (10) verläuft, ein Randpunkt (20) ermittelt wird, dessen Abstand von dem berührten Punkt als ein Wert bestimmt wird, der dem Quotienten $(L(p,\vartheta))$ aus dem zum jeweiligen Strahlenweg gehörenden Absorptionswert$(Q(p,\vartheta))$ und einem vorgewählten, mittleren Absorptionskoeffizienten $(\bar{\mu})$ entspricht.

2.    Verfahren nach Anspruch 1, dadurch gekennzeichnet, daß auf den durch die so ermittelten Randpunkte (20) hindurchlaufenden Strahlenwegen (11), ausgehend von den Randpunkten in Richtung des Körpers (4) jeweils ein weiterer Randpunkt ermittelt wird, dessen Abstand von dem ursprünglichen Randpunkt als ein Wert bestimmt wird, der dem Quotienten $(L(p,\vartheta))$ aus dem zum jeweiligen Strahlenweg gehörenden Absorptionswert $(Q(p,\vartheta))$ und einem vorgewählten, mittleren Absorptionskoeffizienten $(\mu)$ entspricht.

3.   Verfahren nach Anspruch 1, dadurch gekennzeichnet, daß zur Ermittlung verbesserter Randpunkte vom Zentrum des Untersuchungsbereichs (10) ausgehende und bis zu den Randpunkten (20) weisende Vektoren (A(r, φ)) in vorgewählten Winkelbereichen (Δφ) gemittelt werden.

4.   Vorrichtung zur Durchführung des Verfahrens nach einem oder mehreren der Ansprüche 1 bis 3 unter Verwendung wenigstens einer Strahlenquelle 1, deren Strahlung den auf einem Untersuchungstisch liegenden Körper im Untersuchungsbereich in unterschiedlichen in der Ebene liegenden Richtungen auf jeweils einer Anzahl wenigstens annähernd parallel liegender Strahlenwege vollständig durchstrahlt, wobei die Strahlung zur Aufnahme von Meßwerten (I(p,ϑ)) auf eine Detektoranordnung trifft, welche jenseits des Körpers angeordnet ist, sowie einer elektronischen Einheit zur Ermittlung der Absorptionsverteilung der Strahlung mit Hilfe der Meßwerte und der Randpunkte, dadurch gekennzeichnet, daß ein auf dem Patiententisch (13) in definierter Lage befestigbarer, aus starrem Material bestehender Begrenzungskörper (14) vorgesehen ist, auf dem an vorgegebenen Positionen einzelne, wenigstens annähernd in der Ebene liegende Signalgeber (19) angeordnet sind, die bei Berührung mit dem Körper (4) ein Ausgangssignal erzeugen.

5.   Vorrichtung nach Anspruch 4, dadurch gekennzeichnet, daß der Begrenzungskörper (14) aus einer ebenen, rechteckförmigen Platte (15) und zwei Begrenzungsplatten (17) besteht, welche an der Platte an gegenüberliegenden Seiten um jeweils eine Achse (16) schwenkbar angeordnet sind, wobei die Achsen senkrecht zur Ebene verlaufen.

6.   Vorrichtung nach Anspruch 5, dadurch gekennzeichnet, daß auf dem Begrenzungskörper (14) mehrere Zeilen von parallel zur Ebene liegenden Signalgebern (19) angeordnet sind.

7.    Vorrichtung nach einem oder mehreren der Ansprüche 4
bis 6, dadurch gekennzeichnet, daß sich auf dem Begrenzungskörper (14) eine dünne Schaumstoffschicht befindet, auf
die beidseitig eine aus Kunststoff bestehende Folie aufgebracht ist, wobei auf der Innenseite der einen Folie eine
Vielzahl äquidistant angeordneter Längsleiterbahnen (25)
liegt, die senkrecht zur Ebene verlaufen, und auf der
Innenseite der anderen Folie eine Vielzahl äquidistant
liegender Querleiterbahnen (24) angeordnet ist, die senkrecht
zu den Längsleiterbahnen verlaufen, und daß zur Herstellung
eines Kontaktes zwischen den sich kreuzenden Leiterbahnen
die Schaumstoffschicht im Bereich der einzelne Signalgeber (19) darstellenden Kreuzungspunkte der Leiterbahnen
mit Löchern versehen ist.

8.    Vorrichtung nach Anspruch 7, dadurch gekennzeichnet,
daß die Schaumstoffschicht eine druckabhängige elektrische
Leitfähigkeit besitzt.

9.    Vorrichtung nach Anspruch 7, dadurch gekennzeichnet,
daß die Längs-(25) bzw. Querleiterbahnen (24) auf die
Folieninnenseiten aufgedampft sind und vorzugsweise aus
einem Röntgenstrahlung nur wenig absorbierenden Metall
bestehen.

10.   Vorrichtung zur Durchführung des Verfahrens nach
einem oder mehreren der Ansprüche 1 bis 3 unter Verwendung wenigstens einer Strahlenquelle 1, deren Strahlung
den auf einem Untersuchungstisch liegenden Körper im Untersuchungsbereich in unterschiedlichen in der Ebene liegenden
Richtungen auf jeweils einer Anzahl wenigstens annähernd
parallel liegender Strahlenwege vollständig durchstrahlt,
wobei die Strahlung zur Aufnahme von Meßwerten ($I(p,\vartheta)$)
auf eine Detektoranordnung trifft, welche jenseits des
Körpers angeordnet ist, sowie einer elektronischen Einheit
zur Ermittlung der Absorptionsverteilung der Strahlung mit Hilfe der Meßwerte und der Randpunkte, dadurch gekennzeichnet, daß ein auf dem Patiententisch (13) in definierter
Position befestigbarer, aus starrem Material bestehender
Begrenzungskörper (14) mit vorgegebener Geometrie gesehen
ist, der mit einer flexiblen Folie (56) überdeckt ist,
und daß sich zwischen der Folie und dem Begrenzungskörper
eine Flüssigkeit (57) befindet, deren Absorptionskoeffizient
wenigstens annähernd dem des Körpers (4) entspricht.

11.   Vorrichtung nach Anspruch 10, dadurch gekennzeichnet,
daß der Begrenzungskörper (14) aus einer ebenen, rechteckförmigen Auflageplatte (53) und zwei Seitenplatten (54)
besteht, welche an der Auflageplatte an zwei gegenüberliegenden Seiten um jeweils eine Achse (55) schwenkbar
angeordnet sind, wobei die Achsen senkrecht zur Ebene
verlaufen.

12.   Vorrichtung nach Anspruch 4 oder 10, dadurch gekennzeichnet, daß der Begrenzungskörper (14) eine vorgegebene
Geometrie besitzt, die wenigstens näherungsweise der der
in der Ebene liegenden Querschnittsform des zu untersuchenden
Körpers (4) entspricht.

13.   Vorrichtung nach einem oder mehreren der Ansprüche 4
bis 12 unter Verwendung einer Logarithmiereinheit, welche
aus den erhaltenen Meßwerten $(I(p,\vartheta))$ Absorptionswerte
$(Q(p,\vartheta))$ berechnet, die mit einem Datenspeicher verbunden
ist, in dem die Absorptionswerte gespeichert sind, und
der ferner mit einem Zentralrechner und einem Monitor
zur Berechnung und Darstellung der Absorptionsverteilung
$(\mu(x,y))$ verbunden ist, dadurch gekennzeichnet, daß ein
weiterer Speicher (42) vorgesehen ist, in dem die Berührungspunkte $(x_i, y_i, x_i(\alpha), y_i(\alpha))$ der vom Körper (4) berührten
Signalgeber (19) gespeichert sind, und dessen Ausgang sowie
der Ausgang des Datenspeichers (44) mit dem Eingang einer
ersten Recheneinheit (47) verbunden ist, die die Absorptionswerte $(Q(p,\vartheta))$ für diejenigen Strahlenwege $(p,\vartheta)$ aufsucht,

die durch die im Speicher (42) gespeicherten Berührungspunkte verlaufen, daß mit der ersten Recheneinheit eine
zweite Recheneinheit (48) zur Berechnung der Quotienten
$(L(p,\vartheta))$ und mit dieser eine dritte Recheneinheit (49)
zur Berechnung der Randpunkte (20) verbunden ist, deren
Ausgang mit dem Eingang der ersten Recheneinheit zur Ermittlung weiterer Randpunkte verbunden ist, daß mit der
dritten Recheneinheit ferner eine vierte Recheneinheit
(50) zur Erzeugung verbesserter Randpunkte und mit dieser
eine fünfte Recheneinheit (51) zur Berechnung von Absorptionsdaten $(\overline{Q}(p,\vartheta))$ für den Körperbereich, der nicht vollständig
von Strahlung durchsetzt wird, verbunden ist, deren Ausgang
am Eingang des Datenspeichers (44) angeschlossen ist.

Fig.1

Fig.2

**Fig.3**

**Fig.4**

Fig.5

Fig.6

**Fig.7**

$P(r,\varphi)$

11

$L(p,\vartheta)$   $A(r,\varphi)$

X

$(Xi(\alpha),Yi(\alpha))$   Y

$r(\varphi)$   20   52

20

$A(r,\varphi)$

$\varphi$

$\Delta\varphi$

13

56

14

54   53   54

60

21   59

**Fig.8**   **Fig.11**

56   57

14

54   55   53   13   55   54

**Fig.9**

4

54   56   57   14
58   55   53   13   55   58   57   54

**Fig.10**

Europäisches
Patentamt

**EUROPÄISCHER RECHERCHENBERICHT**

EP 79200578.7

| Kategorie | Kennzeichnung des Dokuments mit Angabe, soweit erforderlich, der maßgeblichen Teile | betrifft Anspruch |
|---|---|---|
| | **EINSCHLÄGIGE DOKUMENTE** | |
| D,P | DE - A1 - 2 802 593 (PHILIPS)<br><br>÷ Gesamt + (26.Juli 1979)<br><br>-- | 1-3 |
| | CH - A - 537 734 (BROWN, BOVERIE & CIE.)<br><br>+ Spalte 2, Zeilen 39-44,<br>Spalte 3, Zeilen 28-40,<br>Spalte 4, Zeilen 36-38 +<br><br>-- | 1,4-9 |
| | DE - B - 1 949 765 (SIEMENS)<br><br>+ Spalte 3, Zeilen 49-63,<br>Figur 1 +<br><br>-- | 1,4,5,<br>10-12 |
| | DE - B2 - 2 439 847 (EMI)<br><br>÷ Gesamt +<br><br>& US - A - 4 076 985<br><br>-- | 1,4,10,<br>13 |
| | DE - A1 - 2 503 978 (EMI)<br><br>+ Gesamt +<br><br>-- | 1,4,10,<br>13 |
| | DE - A - 2 609 925 (PHILIPS)<br><br>+ Seite 14 +<br><br>---- | 1 |

**KLASSIFIKATION DER ANMELDUNG (Int.Cl.)** 3

A 61 B 6/00
G 01 N 23/06

**RECHERCHIERTE SACHGEBIETE (Int. Cl.)** 3

A 61 B 6/00
G 01 N 23/00
G 01 T 1/00
G 03 B 41/00
H 05 G 1/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 07-01-1980 | KARLICEK |

EPA form 1503.1 06.78